# EUROPEAN PATENT APPLICATION

(11) **EP 0 779 081 A2**
(43) Date of publication of application: **18.06.1997**
(21) Application number: 96119382.8
(22) Date of filing: 03.12.1996
(51) Int. Cl.: A61N 5/10

(54) **Charged particle beam apparatus and method of operating the same**

(30) Priority: 11.12.1995 JP 321427/95
(71) Applicant: HITACHI, LTD., Chiyoda-ku, Tokyo 100 (JP)
(72) Inventor: Hiramoto, Kazuo, Hitachiota-shi (JP); Tadokoro, Masahiro, Hitachiota-shi (JP); Norimine, Tetsuro, Hitachi-shi (JP)
(74) Representative: Strehl Schübel-Hopf Groening & Partner

(57) **Abstract**

A charged particle accelerator (100) has a switching device (166) for switching on and off the extraction of a charged particle beam, an irradiation unit (110) has electromagnets (220, 221) for setting an irradiation point of the charged particle beam to be irradiated on an irradiation target, and a control unit (130) controls the switching device to cause it to switch on and off the beam extraction and the electromagnets (220, 221) to cause them to change the irradiation point. When the switching device (166) switches on the extraction of the charged particle beam circulating through the charged particle accelerator (100) into the irradiation unit (100), the charged particle beam is irradiated on the irradiation target in the irradiation unit. When the switching device switches off the beam extraction from the charged particle accelerator into the irradiation unit, the irradiation of the charged particle beam on the irradiation target is also stopped. Accordingly, when the control unit (130) controls the switching device to cause it to switch on and off the beam extraction, the irradiation on the irradiation target can also be switched on and off. Since the electromagnets (220, 221) set the irradiation point of the charged particle beam in the irradiation unit (110), the charged particle beam extracted from the charged particle accelerator into the irradiation unit can be irradiated on the irradiation target. Since the control unit (130) controls the electromagnets (220, 221) to cause them to change the irradiation point, the charged particle beam can be irradiated on another irradiation point of the irradiation target. Even when the irradiation target has a complicated shape, the irradiation target can be irradiated with high accuracy through simplified control. The irradiation is continued until a target of irradiation dose is reached and therefore, even when the beam intensity changes temporally, the irradiation target can be irradiated with uniform beam density.

## Description

### BACKGROUND OF THE INVENTION

The present invention relates to a charged particle beam apparatus which utilizes a charged particle beam for cancer therapy and diagnosis of an affected part.

A technique of extracting a charged particle beam circulating through a charged particle accelerator on the basis of a diffusion resonance extraction method to utilize the charged particle beam extracted from the charged particle accelerator for medical treatment is described in JP-A-5-198397.

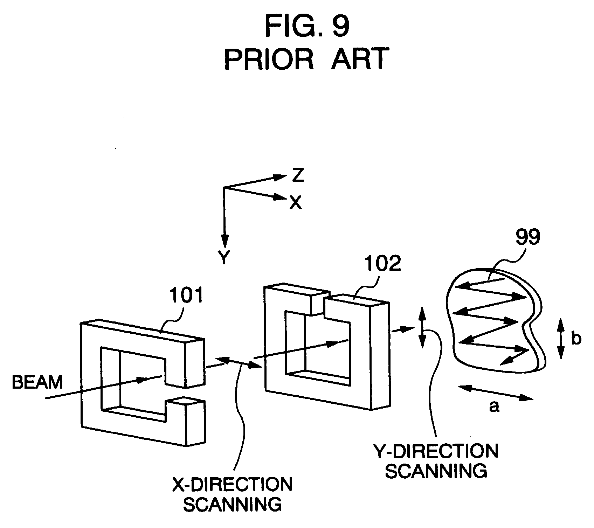

A conventional charged particle beam apparatus is illustrated in Fig. 45 of Rev. Sci. Instrum., Vol. 64, No.8, August, 1993, p.2088. This prior art will be described with reference to Fig. 9.

In Fig. 9, a charged particle beam travels in z direction. When temporally changing currents are passed through an x-direction scanning electromagnet 101 and a y-direction electromagnet 102, magnetic fields generated by these electromagnets are also changed temporally and the charged particle beam is scanned in the x direction (horizontal direction) and in the y direction (vertical direction). In Fig. 9, the number of x-direction scanning (reciprocation) operations per unit time is large and the number of y-direction scanning operations is small so that an irradiation field 99 may be formed. Widths a and b of the irradiation field 99 are determined by maximum currents of the x-direction scanning electromagnet 101 and y-direction scanning electromagnet 102, respectively.

But when an irradiation target has a complicated shape, the scanning widths and scanning speeds in the x and y directions must be changed while the charged particle beam being irradiated in order that the charged particle beam can be irradiated on the irradiation target with high accuracy and with uniform beam density, and hence control of currents supplied to the x-direction scanning electromagnet 101 and
y-direction scanning electromagnet 102 becomes very complicated.

### SUMMARY OF THE INVENTION

An object of the present invention is to provide a charged particle beam apparatus which can irradiate a charged particle beam on an irradiation target with high accuracy and with uniform beam density through simplified control even when the irradiation target has a complicated shape, and a method of operating the apparatus.

To accomplish the above object, according to the present invention, a charged particle accelerator has switching means for switching on and off the extraction of a charged particle beam, an irradiation unit has electromagnets for setting an irradiation point of the charged particle beam to be irradiated on an irradiation target, and a control unit controls the switching means to cause it to switch on and off the beam extraction and the electromagnets to cause them to change the irradiation point.

In the present invention, when the switching means switches on the extraction of the charged particle beam circulating through the charged particle accelerator into the irradiation unit, the charged particle beam is irradiated on the irradiation target in the irradiation unit. When the switching means switches off the beam extraction from the charged particle accelerator into the irradiation unit, the irradiation of the charged particle beam on the irradiation target is also stopped. Accordingly, when the control unit controls the switching means to cause it to switch on and off the beam extraction, the irradiation on the irradiation target can also be switched on and off. Since the electromagnets set the irradiation point of the charged particle beam in the irradiation unit, the charged particle beam extracted from the charged particle accelerator into the irradiation unit can be irradiated on the irradiation target. Since the control unit controls the electromagnets to cause them to change the irradiation point, the charged particle beam can be irradiated on another irradiation point of the irradiation target.

Further, in the present invention, an irradiation dose target setting unit divides the irradiation target into a plurality of irradiation regions and determines targets of irradiation dose at individual irradiation regions, an irradiation dose measuring unit measures irradiation doses of the charged particle beam at the individual irradiation regions, and the control unit controls the switching means on the basis of the targets of irradiation dose and the irradiation doses measured by the irradiation dose measuring unit. Since the control unit controls the electromagnets such that the charged particle beam is irradiated on each irradiation region while the irradiation point being changed, the charged particle beam can be irradiated with high accuracy and with uniform beam density even when the irradiation target has a complicated shape. Further, since the control unit causes the irradiation to be continued until the irradiation dose at the irradiation region reaches the target of irradiation dose, the irradiation target can be irradiated with uniform beam density even when the intensity of the charged particle beam changes temporally.

When the switching means is a radio frequency applying unit for applying to the charged particle beam a radio frequency electromagnetic field containing a frequency of betatron oscillation of the charged particle beam circulating through the charged particle accelerator, the amplitude of betatron oscillation of the charged particle beam increases under the application of the radio frequency electromagnetic field to exceed a stability limit of resonance while the betatron oscillation of the charged particle beam circulating through the charged particle accelerator is brought into a resonance state, and the charged particle beam is extracted from the charged particle accelerator. This method for extraction is called a diffusion resonance extraction method and by using the diffusion resonance extraction method, constant extraction of the charged particle beam can be ensured to permit the charged particle beam to be irradiated with uniform beam density.

Further, in the cancer therapy based on the charged particle beam, energy of the charged particle beam to be irradiated must be changed in accordance with a depth of the irradiation target. To meet this requirement, the extracted charged particle beam is irradiated while being changed in energy by either changing energy of the charged particle beam circulating through the charged particle accelerator during the phase of acceleration or placing a plate-like material such as graphite at a site in the irradiation unit through which the charged particle beam passes.

### BRIEF DESCRIPTION OF THE DRAWINGS

Fig. 1 is a schematic diagram showing a first embodiment of a charged particle beam apparatus of the present invention.

Fig. 2 is a graph showing an example of the relation between depth of an affected part and irradiation dose of an ion beam.

Fig. 3 is a perspective view showing a first embodiment of layers and irradiation regions.

Fig. 4 is a block diagram of an operation unit.

Fig. 5 is a flow chart showing a first embodiment of a method of operating the charged particle beam apparatus.

Fig. 6 is a diagram showing range shifters.

Fig. 7 is a perspective view showing a second embodiment of irradiation regions.

Fig. 8 is a flow chart showing a second embodiment of the method of operating the charged particle beam apparatus.

Fig. 9 is a schematic perspective view showing a conventional charged particle beam apparatus.

### DESCRIPTION OF THE PREFERRED EMBODIMENTS

### (Embodiment 1)

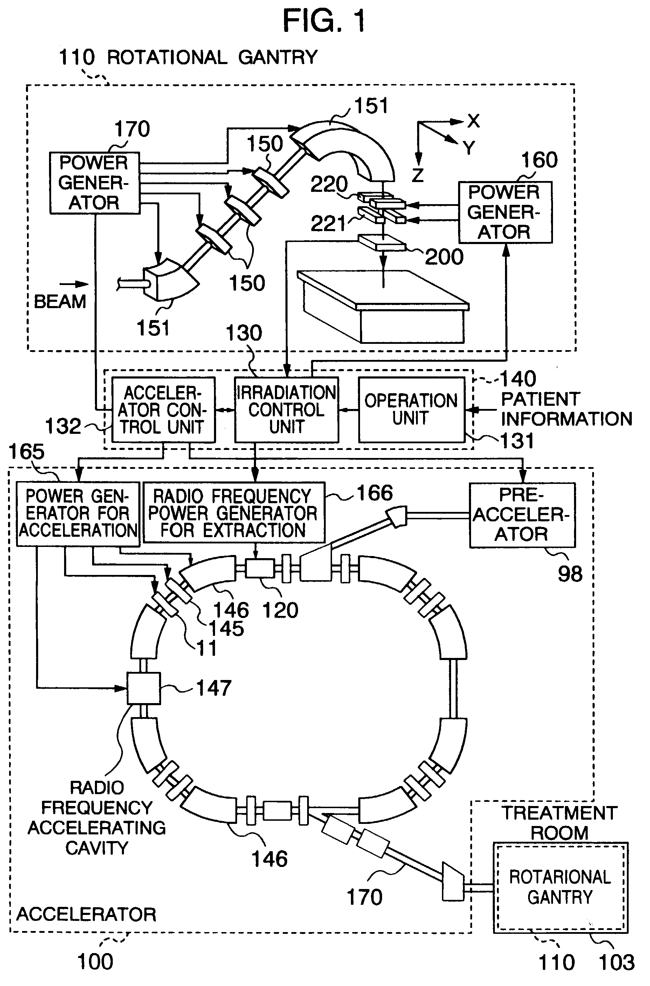

Referring now to Fig. 1, a first embodiment of a charged particle beam apparatus according to the present invention will be described.

The charged particle beam apparatus of the present embodiment mainly includes a pre-accelerator 98, an accelerator of synchrotron type 100, a rotational gantry 110 and a controller group 140. Ions of low energy from the pre-accelerator 98 are injected into the accelerator 100 and they are accelerated by the accelerator 100 and then extracted to the rotational gantry 110 inside a treatment room 103 so that an ion beam may be used for medical treatment.

Main components constituting the accelerator 100 will be described. The accelerator 100 is an accelerator utilizing the diffusion resonance extraction method for beam extraction in which betatron oscillation of a charged particle beam circulating through the accelerator 100 is brought into a resonance state and a radio frequency electromagnetic field is applied to the circulating charged particle beam to increase the betatron oscillation thereof to thereby ensure that a stability limit of resonance can be exceeded and the charged particle beam can be extracted from the accelerator.

The accelerator 100 includes a bending electromagnet 146 for bending the circulating charged particle beam, a radio frequency accelerating cavity 147 for applying energy to the circulating charged particle beam, a quadrupole electromagnet 145 and a multipole electromagnet 11 for magnetic fields to the circulating charged particle beam to generate the stability limit of the resonance of the betatron oscillation, and a radio frequency applying unit for extraction 120 which applies a radio frequency to the circulating charged particle beam to increase the betatron oscillation. The accelerator 100 further includes a power generator for acceleration 165 which supplies currents to the bending electromagnet 146, quadrupole electromagnet 145 and multipole electromagnet 11 and which supplies electric power to the radio frequency accelerating cavity 147, and a radio frequency power generator for extraction 166 which supplies electric power to the radio frequency applying unit for extraction 120.

The rotational gantry 110 will be described. The rotational gantry 110 includes quadrupole electromagnets 150 and bending electromagnets 151 which are adapted to transport a beam extracted from the accelerator 100 to an irradiation target, and a power generator 170 for supplying currents to the quadrupole electromagnets 150 and bending electromagnets 151.

The rotational gantry 110 further includes electromagnets 220 and 221 which are provided downstream of one of the bending electromagnets 151 and operative to deflect the extracted beam in x and y directions. Here, the x direction is parallel to the bending plane of the one bending electromagnet 151 and the y direction is vertical to the bending plane of the one bending electromagnet 151. The electromagnets 220 and 221 are connected with a power generator 160 for supplying currents to them. An irradiation dose monitor 200 for measuring irradiation dose distribution of the beam is provided downstream of the electromagnets 220 and 221 and immediately before a patient standing for the irradiation target.

The controller group 140 will be described. The controller group 140 includes an irradiation control unit 130, an operation unit 131 and an accelerator control unit 132.

The irradiation control unit 130 is a control unit for controlling the extraction of the charged particle beam from the accelerator 100 into the rotational gantry 110. Since the charged particle beam extracted from the accelerator 100 into the rotational gantry 110 is irradiated on the irradiation target, controlling the extraction from the accelerator 100 is to control the irradiation of the charged particle beam on an affected part.

The operation unit 131 is a unit for determining data necessary for the irradiation control unit 130 to control the irradiation of the charged particle beam on the affected part.

The accelerator control unit 132 is a unit for controlling the extraction of the charged particle beam from the pre-accelerator 98 into the accelerator 100, the acceleration of the charged particle beam circulating through the accelerator 100 and the transport of the charged particle beam in the rotational gantry 110.

The role of the operation unit 131 will first be described and a method of operating the charged particle beam apparatus by means of the irradiation control unit 130 and accelerator control unit 132 will then be described.

Affected part information such as shape and depth of an affected part and a necessary irradiation dose R is inputted to the operation unit 131 by an operator. On the basis of the inputted affected part information, the operation unit 131 calculates and determines an irradiation region, energy of the charge particle beam to be irradiated on the affected part and the magnitude of currents to be supplied to the electromagnets 220 and 221.

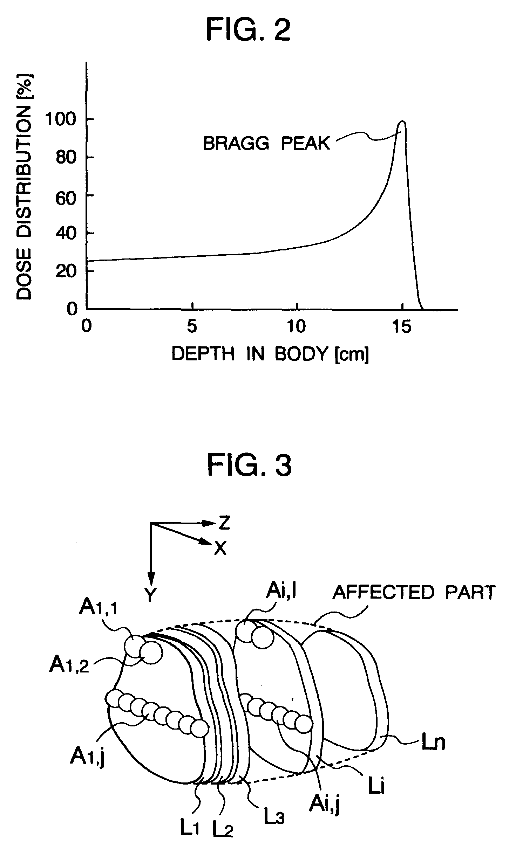

Here, the relation between depth of the affected part and energy of the charged particle beam will be described. Fig. 2 shows an example of the relation between depth in a body and irradiation dose of the charged particle beam. A peak of the irradiation dose shown in Fig. 2 is called a Bragg peak. The irradiation of the charged particle beam is effected at a position of the Bragg peak. The Bragg black peak position changes with energy of the charged particle beam. Accordingly, by changing energy in accordance with depths in the affected part, the charged particle beam can be irradiated on the whole of the affected part having a thickness in the depth direction.

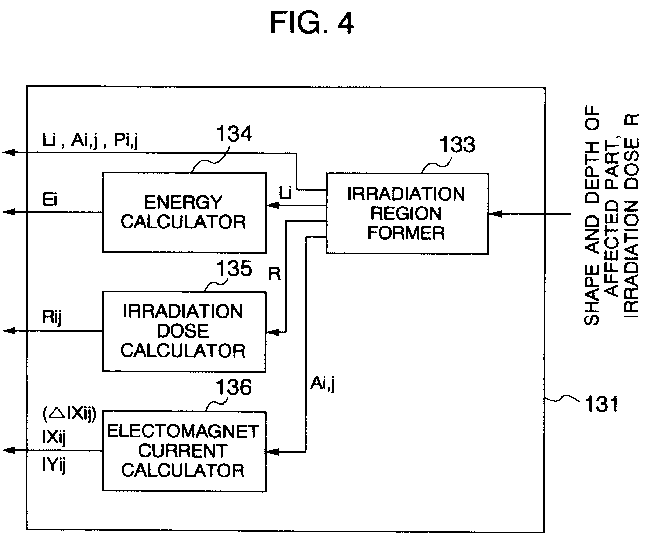

The operation unit 131 is shown in Fig. 4.

An irradiation region former 133 of the operation unit 131 divides the affected part into a plurality of layers, as generally designated by Li (i=1, 2, ··· N), in the depth direction on the basis of the inputted affected part information, as shown in Fig. 3. An energy calculator 134 determines beam energy levels, as generally designated by Ei, suitable for irradiation in accordance with depths of the individual layers.

Further, the irradiation region former 133 determines a plurality of irradiation regions as generally designated by Ai,j (i=1, 2 ···· N, j=1, 2 ··· M), center points Pi,j of the irradiation regions Ai,j and coordinate values (xij, yij) of the center points in accordance with shapes of the individual layers Li. Since the intensity of the charged particle beam is spatially distributed pursuant to Gauss distribution, the operation unit 131 determines the individual irradiation regions Ai,j and their center points Pi,j on the basis of a size of the charged particle beam in such a manner that an irradiation region Ai,j somewhat overlaps an adjacent irradiation region. An irradiation dose calculator 135 determines targets of irradiation dose at the individual center points Pi,j on the basis of the necessary irradiation dose R.

An electromagnet current calculator 136 determines currents IXij and IYij supplied to the electromagnets 220 and 221 in order that the center of the charged particle beam matches the individual center points Pi,j.

The operation unit 131 delivers to the irradiation control unit 130 beam energy Ei, individual irradiation regions Ai,j, center points Pi,j, coordinate values (xij, yij) of the center points Pi,j, targets of irradiation dose Ri,j and currents IXij and IYij which are determined in respect of the individual layers Li.

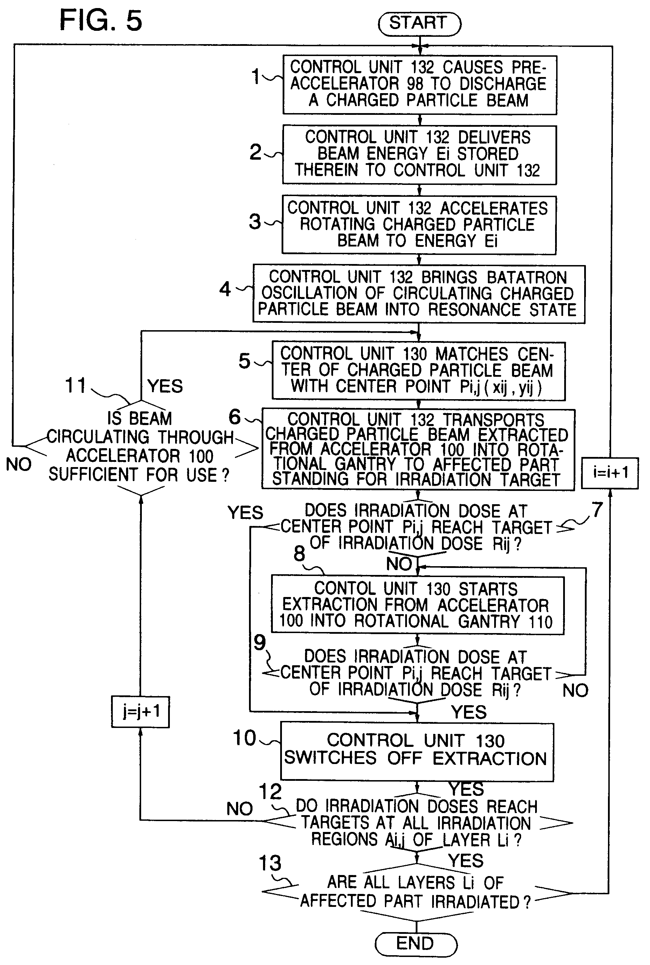

A method of operating the charged particle beam apparatus of the present embodiment is shown in Fig. 5.
(1) The accelerator control unit 132 controls the pre-accelerator 98 to cause it to eject a charged particle beam.
(2) The irradiation control unit 130 delivers a beam energy level Ei stored therein to the accelerator control unit 132.
(3) The accelerator control unit 132 supplies currents to the bending electromagnet 146, quadrupole electromagnet 145 and multipole electromagnet 11 in order to accelerate the circulating charged particle beam to the energy level Ei and control the power generator for accelerator 165 to cause it to supply electric power to the radio frequency accelerating cavity 147.
(4) When the circulating charged particle beam is accelerated to the energy level Ei, the accelerator control unit 132 controls the power generator for accelerator 165 to cause it to supply currents to the quadrupole electromagnet 145 and multipole electromagnet 11 in order to generate the stability limit of the resonance of betatron oscillation .
   When the electric power is supplied to the radio frequency applying unit for extraction 120, the betatron oscillation amplitude of the circulating charged particle beam increases and this results in the resonance state of the betatron oscillation for the charged particle beam outside the stability limit.
(5) The irradiation control unit 130 controls the power generator 160 to cause it to supply currents IXij and IYij to the electromagnets 220 and 221 in order that the center of the charged particle beam matches an optional center point Pi,j.
(6) The accelerator control unit 132 controls the power generator 170 to cause it supply currents to the quadrupole electromagnets 150 and bending electromagnets 151 in order that the charged particle beam extracted from the accelerator 100 into the rotational gantry 110 is transported to an affected part standing for the irradiation target.
(7) The irradiation control unit 130 compares a target of irradiation dose Rij with an irradiation dose at the particular center point Pi,j measured by the irradiation dose monitor 200.
(8) When the irradiation dose at the particular center point Pi,j does not reach the target of irradiation dose Rij, the irradiation control unit 130 controls the radio frequency power generator for extraction 166 to cause it to supply electric power to the radio frequency applying unit for extraction 120 in order to start the extraction from the accelerator 100 to the rotational gantry 110.
   When electric power is supplied to the radio frequency applying unit for extraction 120, a radio frequency electromagnetic field is applied to the circulating charged particle beam to increase the betatron oscillation amplitude of the circulating charged particle beam. When the betatron oscillation amplitude is increased until a stability limit of resonance of the betatron oscillation is exceeded, the charged particle beam is extracted from the accelerator 100 into the rotational gantry 110. In the rotational gantry 110, the charged particle beam is irradiated on an optional irradiation region Ai,j.
(9) The irradiation control unit 130 compares a target of irradiation dose Rij with an irradiation dose at another center point Pij measured by the irradiation dose monitor 200. When the irradiation dose at the center point Pi,j does not reach the target of irradiation dose Rij, the extraction is continued.
(10) When the irradiation dose at the different center point Pi,j reaches the target of irradiation dose Rij, the irradiation control unit 130 controls the radio frequency power generator for extraction 166 to cause it to switch off the extraction. Then the irradiation control unit 130 controls the power generator 160 such that the center of the charged particle beam matches a center point Pi,j+1 of next irradiation region Ai,j+1.
(11) When the beam circulating through the accelerator 100 is sufficient for use at the time that irradiation on the irradiation region Ai,j shifts to irradiation on the irradiation region Ai,j+1, the operation is carried starting with step (5) but when the beam intensity and the extraction time are insufficient, the operation is carried out starting with step (1) for the purpose of replenishing the charged particle beam.
(12) When the irradiation doses reach the targets at all irradiation regions Ai,j of an optional layer Li, the operation starting from step (1) is carried out for next layer Li+1 and all irradiation regions Ai+1,j are irradiated in a manner similar to that in the case of the layer Li.
(13) When irradiation on all of the layers Li of the affected part are completed, the operation of the charged particle beam apparatus ends.

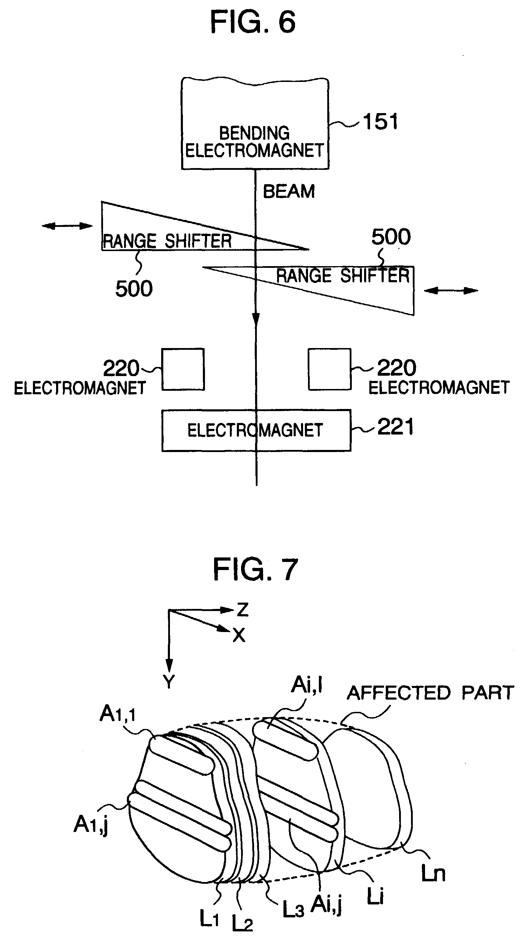

While in the present embodiment the energy of the charged particle beam is set to Ei in the accelerator 100, the energy of the charged particle beam may be changed in the rotational gantry 110. For example, range shifters 500 as shown in Fig. 6 are disposed immediately before the electromagnet for beam irradiation point setting 220. Then, by causing the irradiation control unit 130 to drive the range shifters 500 so as to change their thickness, energy of the charged particle beam transmitting through the range shifters 500 can be changed.

According to the present embodiment, even when the irradiation target has a complicated shape, the affected part can be irradiated with high accuracy. Further, since the irradiation is continued until the irradiation dose reaches a target, the affected part can be irradiated with uniform beam density even when the beam intensity changes with time.

### (Embodiment 2)

Next, a second embodiment of the present invention will be described. Component construction of the present embodiment is similar to that of the first embodiment. In the present embodiment, however, each layer Li of an affected part is not divided into irradiation regions in the x direction but is divided only in the y direction as shown in Fig. 7. In other words, irradiation regions Ai,j are each wide in the x direction. Another irradiation region Ai,j is irradiated by changing the strength of a magnetic field generated by the electromagnet 220 to scan the charged particle beam in the x direction.

The operation unit 131 determines magnitude ΔIXij necessary for changing the magnetic field strength of the electromagnet 220 on the basis of an extent of each region Ai,j in the x direction. As in the case of embodiment 1, the operation unit determines the beam energy Ei, individual irradiation regions Ai,j, their center points Pi,j (xij, yij), targets of irradiation dose Rij, and currents IXij and IYij in respect of the individual layers Li and delivers them, together with the ΔIXij, to the irradiation control unit 130.

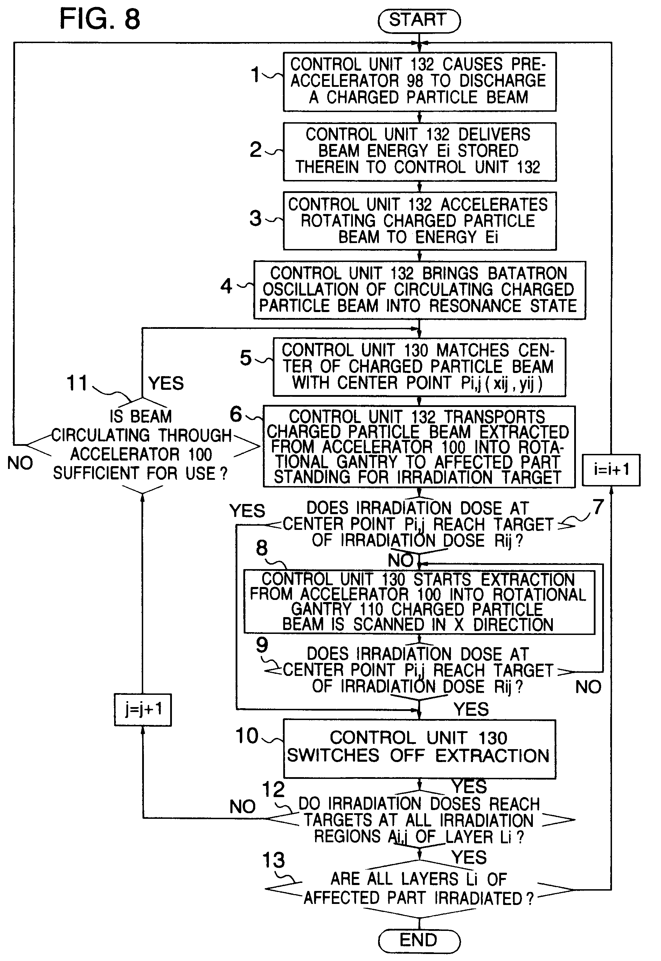

A method of operating the charged particle beam apparatus of the present embodiment is shown in Fig. 8. Excepting step (8), operation steps are the same as those of the first embodiment.

In step (8), the irradiation control unit 130 controls the radio frequency power generator for extraction 166 to cause it to supply electric power to the radio frequency applying unit for extraction 120 in order that the extraction from the accelerator 100 into the rotational gantry 110 is started and besides, controls the power generator 160 such that current IXij to the electromagnet 220 changes within a range of ΔIXij to cause the charged particle beam to be irradiated while being scanned in the x direction.

In the present embodiment, too, the energy of the charged particle beam may be changed in the rotational gantry 110 by using the range shifters 500 as described in connection with embodiment 1.

While in the present embodiment the strength of the magnetic field generated by the electromagnet 220 is changed to scan the charged particle beam in the x direction and irradiate it on the irradiation region Ai,j, the charged particle beam may be irradiated while being scanned in the y direction by changing the strength of the magnetic field generated by the electromagnet 221.

## Claims

1. A charged particle beam apparatus comprising a circular charged particle accelerator (100) and an irradiation unit (110) for irradiating a charged particle beam supplied from said charged particle accelerator on an irradiation target,
wherein said charged particle accelerator (100) has switching means (130, 166) for switching on and off an extraction of said charged particle beam, said irradiation unit (110) has electromagnets (220, 221) for setting an irradiation point of said charged particle beam to be irradiated on said irradiation target, and a control unit (130) is provided which controls said switching means to cause it to switch on and off the extraction of said charged particle beam and said electromagnets (220, 221) to cause them to change said irradiation point.

2. A charged particle beam apparatus according to claim 1 further comprising an irradiation dose target setting unit (131) for dividing said irradiation target into a plurality of irradiation regions (Ai,j) and determining targets of irradiation dose at individual irradiation regions, and an irradiation dose measuring unit (200) for measuring irradiation doses of said charged particle beam at the individual irradiation regions,
wherein said control unit (130) controls said switching means on the basis of the targets of irradiation dose and the irradiation doses measured by said irradiation dose measuring unit (200).

3. A charged particle beam apparatus according to claim 1, wherein said switching means is a radio frequency applying unit (166) for applying to said charged particle beam a radio frequency electromagnetic field containing a frequency of betatron oscillation of said charged particle beam circulating through said charged particle accelerator (100).

4. A charged particle beam apparatus according to claim 3 further comprising energy changing means (500) for changing energy of said charged particle beam to be irradiated on said irradiation target.

5. A charged particle beam apparatus according to claim 4, wherein said energy changing means (500) is disposed in said irradiation unit (110).

6. A method of operating the charged particle beam apparatus as recited in claim 1, comprising the steps of:
switching on and off an extraction of a charged particle beam;
setting individual irradiation points; and
changing an optional irradiation point to another.

7. A method of operating the charged particle beam apparatus as recited in claim 2,
wherein a plurality of irradiation regions (Ai,j) are determined on an irradiation target, targets of irradiation dose at individual irradiation regions are determined, irradiation points for each of the individual irradiation regions are set, a charged particle beam is extracted from a circular charged particle accelerator (100), an irradiation dose of said charged particle beam at the optional irradiation region is measured, an extraction of said charged particle beam is switched off on the basis of the target of irradiation dose and the measured irradiation dose, and the irradiation point is changed from said optional point to another.

8. A method of operating the charged particle beam apparatus according to claim 7,
wherein a radio frequency electromagnetic field is applied to said charged particle beam to switch on the extraction of the charged particle beam, and the application of said radio frequency electromagnetic field to said charged particle beam is stopped to switch off the extraction of the charged particle beam.

9. A method of operating the charged particle beam apparatus according to claim 6 or 7 further comprising the step of changing energy of said charged particle beam.
